# EUROPEAN PATENT APPLICATION

(11) **EP 3 974 332 A1**
(43) Date of publication of application: **30.03.2022**
(21) Application number: 20932098.5
(22) Date of filing: 16.11.2020
(51) Int. Cl.: B65B 55/04, B65B 55/06, B65B 55/10, B65B 31/00, B67C 7/00, A61L 2/00, A61L 2/22

(54) **BEVERAGE BOTTLING PLANT**

(30) Priority: 09.11.2020 RU 2020136750
(71) Applicant: Klinetsky, Evgeny Fedorovich, Moskovskaya oblast, g. Zhukovsky, 140180 (RU)
(72) Inventor: Klinetsky, Evgeny Fedorovich, Moskovskaya oblast, g. Zhukovsky, 140180 (RU)
(74) Representative: Jeck, Jonathan
(86) International application number: PCT/RU2020/000607
(87) International publication number: WO 2021/215959

(57) **Abstract**

The invention relates to devices for filling and closing containers, bottles or other receptacles in a sterilizable environment. The present beverage bottling plant is a sterile non-hermetic isolator, the inside of which is filled with a sterilizing mixture containing hot air and atomized finely dispersed hydrogen peroxide H₂O₂, i.e. hydrogen peroxide vapour. The plant is configured on the principle of a single-shell isolator enclosure and operates continuously. Air extractor casings are arranged at a receptacle inlet and a receptacle outlet, said casings creating controlled movement of a sterilizing medium inside and providing a continuous entrainment effect. The inside walls of a receptacle are treated with hot sterile air directly before the receptacle is filled with a product, and the inside walls of a lid are treated with hot sterile air directly before closure. Directly before filling, the interior of a receptacle is flushed with a jet of hot sterile air to remove peroxide residues. The inside part of the lid is also flushed with a jet of hot sterile air. Flushing with hot sterile air is carried out by nozzles mounted inside the enclosure along the path of the receptacles and lids. The technical result is a more reliable and simple filling and closing process.

## Description

The invention relates to devices for filling and closing containers, bottles or any other containers occurring in a sterilized environment.

In the first plants, containers were filled and capped in non-antiseptic devices, and then the molded containers were moved to a "clean room", at least a filling machine and a capping.

Moreover, the "usual" aseptic filling line provides:
- forming a container starting with a billet of thermoplastic material or a preform;
- chemical sterilization of a molded container or preform;
- filling and capping of a filled container, which must be carried out in a clean room.

A "clean room" is a controlled contamination room that contains all filling devices, including both technological zones in which containers are effectively filled/closed, and auxiliary zones in which the means of moving the filling/capping machine operate.

"Therefore, the main drawback of the "clean room" is represented by its significant volume, where long and expensive sterilization procedures are required, which automatically leads to the complexity of the device and the high cost of its operation. "There is also a significant loss of working fluids, for example, sanitation fluids and sterile air, as well as wear phenomena, for example, filters necessary for air purification, designed to create overpressure in a clean room in order to prevent the ingress of pollutants from the external environment. Another disadvantage of using a "clean room" is associated with difficulties in performing operations to change the container format, maintenance or configuration of machine components due to the risk of contamination that such operations imply. Therefore, access to the "clean room" by the operator is also especially important.

The evolution of aseptic technology has gone towards reducing the volumes that should be sterile.

Therefore, the modern concept of the aseptic filling line provides:
- sterilization of the blank for the container using chemical agents or radiation sterilization;
- aseptic" container forming, starting from the sterilized blank; filling and capping of the filled container, which must be performed in a sterile environment.

A sterilization active zone for filling containers is known (see, patent US No.9296600 B2 IPC B67C 3/00, 3/22, publication dated 29.03.2016), containing the first module that flushes and sterilizes empty containers and delivers sterilized containers to the second module that fills and closes containers in the sterilization active zone using an electron beam sterilization unit.

This system provides for the breakdown of the filling installation into blocks, i. e. the container is sterilized in one block, the lid is sterilized in another, filling and capping in the third. Disinfection is carried out by irradiation, but does not exclude treatment with a sterilizing mixture. This in itself leads to the complexity of the design, maintenance, sealing of the plant.

There is known a filling plant for aseptic liquid filling of bottles (see US Patent No. 7404276 B2 IPC F16J 15/40, 15/42, publication dated 07/29/2008) containing a sterile "clean room" with a given concentration of hydrogen peroxide (H2O2), which is separated from a non-sterile room or space by a siphon seal. The essence of this device consists in separating and sealing two different rooms. A complex liquid barrier and bellows are used, which leads to the complexity of manufacturing and its high cost, as well as to complex maintenance. The essence of this device consists in separating and sealing two different rooms. A complex liquid barrier and bellows are used, which leads to the complexity of manufacturing and its high cost, as well as to complex maintenance. A device for sterile filling with liquids in bottles is known (see application US No. 20090071104 A1 IPC A61L 2/22, B65B 55/10, B67C 7/00, publication dated 03/19/2009) containing a sterilizer for sterilizing the bottle with hydrogen peroxide H2O2 and removing it from the container with sterile air, in addition, devices for filling and closing bottles are provided, and a temperature above the dew point is used to ensure very fast sterilization due to thermally increased reaction rates. The device assumes gas tightness of the entire system, including the sterilization tunnel. This is achieved due to complex sealing. In addition, the chambers where the container is processed and filled are also hermetically separated. All this causes difficulties in the manufacture and operation of the plant.

An aseptic filling and sealing machine is known (see application JP No.2018047944A IPC A61L 2/18, 2/20, B65B 55/04, 55/10, B67C 7/00, publication dated 29.03.2018), including a hermetically sealed container, a heating device for heating sterile air and injection through an air outlet nozzle, as well as a generator of sterilization gas for injection through spray nozzles into a hermetically sealed container.

The invention relates to classical asepsis, with separation of sterilization and bottling zones. It is required to maintain tightness with all the ensuing difficulties in operation and maintenance.

A system of aseptic filling of packages with liquid products is known (see application US No. 20040222224 A1 IPC B67C 7/00, publication dated 11.11.2004), excluding specialized machines and requirements for sterile air spaces of existing aseptic filling systems, providing for the use of membranes over the filling opening of the package, which allows you to capture and maintain sterile contents even when transported in non-sterile air spaces. The operation of the system is based on special flexible membranes that are inserted into the bottle and hold the sterilizing and sterile environment inside the container. The operation of the system is based on special flexible membranes that are inserted into the bottle and hold the sterilizing and sterile environment inside the container. The The advantage of this plant is that you can use conventional, non-aseptic equipment, but there is an additional consumable material that will lead to an increase in the cost of a unit of production, and, in addition, will inconvenience the consumer when opening a drink and, as practice shows, products with inserts are not in demand.

A system for sterilizing bottles with a gaseous sterilizer is known (see, patent US No. 9802726 B2 IPC A61L 2/208, B65 B55/02, 55/10, 55/18, publication dated 31.10.2017), containing a source of a gaseous sterilizer hydraulically connected to injectors moving along with bottles, for decontamination of which evaporated hydrogen peroxide (H2O2) is used. The patented plant is a complex rotary device, since filling with a sterilizing mixture and blowing are carried out simultaneously along with the passage of bottles through the carousels. This undoubtedly causes the high cost and complexity of manufact During sterilization, the injection device is lowered deep into the bottle, which requires precise positioning of the bottle under the injection device. During sterilization, the injection device is lowered deep into the bottle, which requires precise positioning of the bottle under the injection device. Sterilization, blowing of the mixture are located in different chambers, and closed channels between the chambers, as well as a solid body require high-quality sealing of the plant. Expensive filters are used to prepare sterile air.

A device for sterilization is known (see US Patent No. 4797255A IPC A61L 2/20, B65B 55/10, publication dated 10.01.1989), including the creation of hydrogen peroxide transported by air heated to a temperature that is equal to or exceeds the evaporation surface temperature of the object to be sterilized.

This technical solution focuses specifically on the preparation of a mixture of hydrogen peroxide and air, and the goal is to reduce its consumption. Despite this, a plant option is given, where more emphasis is also placed on the disinfection of the container. The injection zone is allocated to a separate chamber, and the blowing zone is not fenced off from the filling zone with the product. In addition, no attention is paid to how a sterile or sterilizing atmosphere is maintained during bottling. In addition, no attention is paid to how a sterile or sterilizing atmosphere is maintained during bottling. Additionally, ultraviolet light is used to disinfect containers. Judging by the drawing and despite the lack of information in the patent description, it can be concluded that such a plant will require high-quality sealing with ventilation.

A device for sterilizing and filling packaging containers is known (see, US Patent No. 6351924 B1 IPC A61L 2/186, B65B 55/10, B67C 7/00, publication dated 05.03.2002), which is exposed to a sterilizing agent during transportation with controlled synchronization in the processing line through various processing stations, which is then removed with sterile hot air without an expensive "clean room" and in a technically simple way.

However, the invention has a number of significant technical disadvantages:
1. The conveyor along which the bottles move has an intermittent drive, the positioning of the bottle under the disinfection station must be very accurate for this plant to be carried out.
2. The absence of sterilization of the lid, however, the patent owner clarifies: by providing a sterile capping agent (applying a sterile closure to the packaging container), a sealing plate is used in this installation. This is an additional consumable, in addition, which is not popular with the consumer.
3. The absence of hydrogen peroxide (H2O2) treatment of transmitting stars, and the bottle is not held by the throat, but a special design is used with special non-standard grips equipped with plates and rods for individual placement and support of each container, which complicates the design of the installation and increases the cost of the bottling process, in addition, disinfection of which is not performed.
4. Since the conveyor is linear, and the increase in plant productivity is achieved by increasing the number of processing stations arranged in a row (in the technical solution there are eight of them, i.e. eight containers can be processed simultaneously), this leads to an increase in the cost of the plant.

In addition, the creators of the technical solution stipulate that in order to achieve this method of disinfection, namely, feeding the sterilizing mixture under high pressure and simultaneously removing it through the ventilation duct (the same with air purging), the container should preferably have the shape of a bottle, since the distribution of the inlet/outlet flows of the mixture is effective only in this case due to geometric features.

The technical result of the proposed solution is to increase the reliability, simplify and reduce the cost of the bottling process by creating a hydrogen peroxide sterilizer of a reasonable structure that provides the required high biological purity of the bottled beverage occurring in a sterilizing environment.

Another advantage of the solution is the creation of an installation for aseptic filling of beverages of a simple design with a stable process of aseptic filling. He closest in terms of technical solution and the achieved result is the device for filling drinks RU 2694 248 C1 MPC A61L 2/00. The essence of the solution is that the beverage bottling plant is implemented using a two-circuit circuit: an internal leaky insulator and an external circuit - the outer walls of the monoblock. The pressure in the inner circuit (insulator) is higher than atmospheric, the pressure between the inner insulator and the outer walls of the monoblock is lower than atmospheric.

The excess pressure in the internal leaky insulator is created by feeding a fine mixture of peroxide and hot air into the insulator, which are used to process containers and lids.

The reduced pressure in the gap between the insulator and the outer walls of the monoblock is achieved due to the hood installed in the roof of the monoblock. However, in order to create control over the maintenance of peroxide vapor in the insulator, more precise settings of the hood are required, since the space between the insulator and the outer walls of the monoblock has significant dimensions. In addition, the two-circuit system significantly complicates the maintenance of the installation (changing the container format, repair, etc.) and, most importantly, requires a more complex system of automatic washing of equipment when changing the product (CIP).

The objective of the claimed installation is to eliminate the shortcomings of analogues and prototype, and the technical result is to increase reliability, simplify the filling and capping process by creating a sterilizer of a reasonable structure that provides the required high biological purity of the bottled beverage occurring in a sterilizing environment. The plant is made in a single-circuit design using a completely new method of exhaust equipment, which allows you to more accurately regulate air flows both inside the installation and in the area of the largest holes in the casing of the installation - at the inlet and outlet of the container, maintaining a high degree of sterility of the product and the safety of the workspace outside the device. The achieved technical result is realized due to the fact that the beverage bottling plant is characterized by the fact that it is a sterile leaky insulator, the inner space of which is filled with a sterilizing mixture including hot air and atomized fine hydrogen peroxide - H2O2 - peroxide vapor, is made according to the principle of a single-circuit insulator housing starting from the entrance of the container into the sterile space of the filling machine and until the exit of the already sealed container with the product, works in continuous mode, at the inlet and outlet of the container there are hoods of the hood, which create the movement of the sterilizing medium inside, while the hoods of the hood provide a permanent effect, with the right flow rate of the hoods, such a movement of the sterilizing medium is created, which eliminates the ingress of peroxide vapor outside through the leaky skin, the air entering through the leaky seals of the space and through the feed channel of the covers immediately mixes with peroxide vapor and becomes sterile, this eliminates the ingress of microorganisms into the sealed container, peroxide vapor inside the monoblock is formed by feeding inside a sprayed fine-dispersed mixture with a high concentration of peroxide for processing incoming containers inside and outside and for processing lids for sealing containers., and also due to the supply of sterile hot air inside to neutralize peroxide on the inner walls of the container and on the inner walls of the lids, the treatment of the inner walls of the container with hot sterile air occurs immediately before filling the container with the product, the treatment of the inner walls of the lids with hot sterile air takes place immediately before capping, finely dispersed sprayed hydrogen peroxide and hot sterile air are supplied through the nozzles, the treatment of the inner walls of the lids with hot sterile air takes place immediately before capping, finely dispersed sprayed hydrogen peroxide and hot sterile air are supplied through nozzles for the treatment of containers and lids the treatment of the inner walls of the lids with hot sterile air takes place immediately before capping, finely dispersed sprayed hydrogen peroxide and hot sterile air are supplied through nozzles for the treatment of containers and lids, the peroxide deposited on the walls of containers and lids kills the remnants of microorganisms, ensures the sterility of the original packaging before bottling and capping, immediately before bottling, the container is purged with a jet of hot sterile air to remove the remnants of peroxide from the inner walls of the container, immediately before closing the container, the inner part of the lids is also purged with a jet of hot sterile air, purging with hot sterile air is carried out by nozzles installed inside on the way of movement of the container and lids, as a result of exposure to hot air, the peroxide deposited on the inner walls of the container and the inner walls of the lid decomposes into water and oxygen, which reduces the concentration of residual peroxide in the product in the sealed container to acceptable value.

The invention is illustrated with graphic materials, a plant for filling drinks of a rotary type is given:

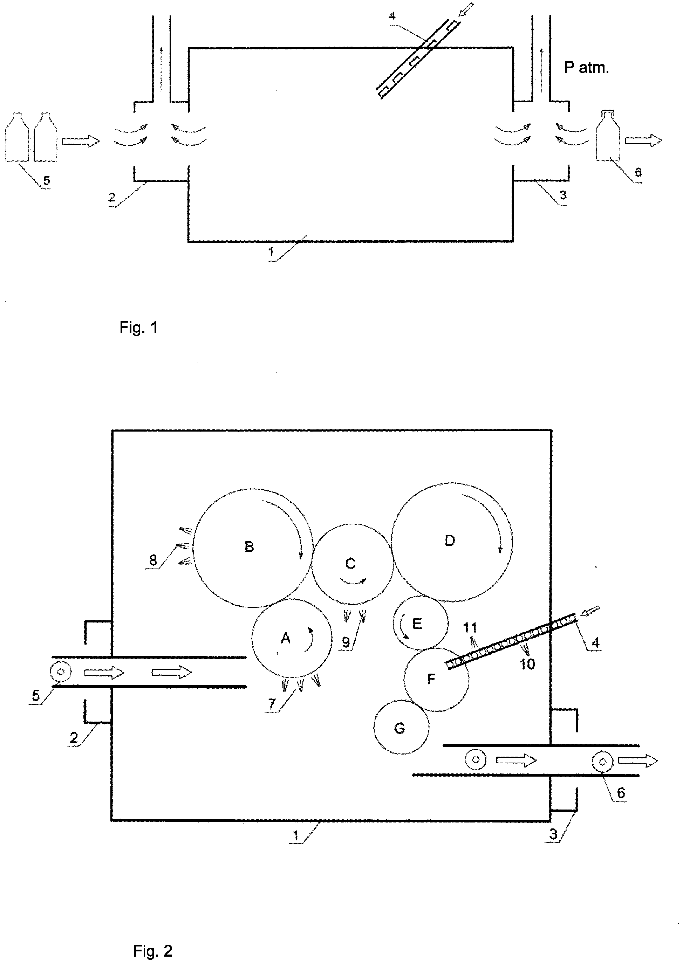

- Fig. 1 -: side view of the plant.
- Fig. 2 -: top view of the plant.

The composition of the proposed object:
- 1.: External walls of the plant (monoblock).
- 2.: Hood at the inlet of the monoblock (arrows show the movement of air flows).
- 3.: Hood at the outlet of the monoblock (arrows show the movement of air flows).
- 4.: Feeding the lids into the monoblock (arrows show the direction of movement of the lids).
- 5.: Feeding empty containers into the monoblock (arrows show the direction of movement of the container).
- 6.: Exit of filled and capped containers from the monoblock (arrows show the direction of movement of the container).
- 7.: Nozzles for processing containers with finely dispersed mixture of hydrogen peroxide on the star A.
- 8.: Nozzles for processing containers with a fine mixture of hydrogen peroxide on a star B.
- 9.: Nozzles for processing containers with hot sterile air on the star C immediately before feeding the container for filling the product produced on the carousel D.
- 10.: Nozzles for processing lids with a fine mixture of hydrogen peroxide at the inlet of the lids into the monoblock.
- 11.: Nozzles for processing containers with hot sterile air immediately before capping the containers produced at the star F.

### Fig. 1.

The filling plant is a relatively closed space in the form of a monoblock 1 with holes for the entry of empty containers through the hood 2 and lids for capping containers 4 and the exit of finished products through the casing 3. The arrows show the direction of movement of the air mixture in the casings and the direction of movement of empty containers, lids and sealed containers.

### Fig. 2.

At the inlet of the monoblock, the container falls on the star A. When the container moves along the star A and then along the star B, the container is treated with a finely dispersed mixture of hydrogen peroxide from the inside and outside with nozzles 7 and 8 to destroy microorganisms on the walls of the container. On star C, immediately before the container is fed to the filling carousel (star D), the inner walls of the container are treated with hot sterile air supplied by nozzles 9 inside the container. On the carousel D, the container is filled with the product. The caps for capping are fed into the monoblock from the outside through a special channel 4 through a hole in the wall of the monoblock. Immediately upon entering the monoblock, the lids are treated with a finely dispersed mixture of hydrogen peroxide 10 and immediately before capping, the inner walls of the lids are treated with hot sterile air supplied by nozzles 11. The sealing of the container is carried out on the star F. The finished products are removed from the monoblock through the star G, and then through the casing 3 to the outside.

The plant works as follows.

The beverage filling unit (monoblock) is a sterile leak-proof insulator, the inner space of which is filled with a sterilizing mixture including hot air and atomized fine hydrogen peroxide (peroxide - H2O2) - peroxide vapor. The plant is carried out on the principle of a single-circuit insulator housing starting from the entrance of the container into the sterile space of the filling machine and until the exit of the already sealed container with the product. The monoblock works in continuous mode. At the inlet and outlet of the container into the monoblock there are hoods of the hood, which create a controlled movement of the sterilizing medium inside the leaky monoblock. The hoods of the monoblock provide a constant ejection effect, thanks to which they suck partially peroxide vapor from the monoblock and partially air from the surrounding space of monoblock. With a properly selected exhaust flow rate, such a movement of the sterilizing medium inside the leaky monoblock is created, which eliminates the ingress of peroxide vapor outside through the leaky lining of the monoblock, the air entering through the leaky seals of the monoblock space and through the feed channel of the lids into the monoblock immediately mixes with peroxide vapor and becomes sterile, this eliminates the ingress of microorganisms into the sealed container. The peroxide vapor inside the monoblock is formed by feeding a sprayed fine mixture with a high concentration of peroxide into the monoblock for processing incoming containers inside and outside and for processing lids for sealing containers, as well as by feeding sterile hot air into the monoblock to neutralize peroxide on the inner walls of the container and on the inner walls of the lids. The treatment of the inner walls of the container with hot sterile air takes place immediately before filling the container with the product, the treatment of the inner walls of the lids with hot sterile air takes place immediately before capping. Finely dispersed atomized hydrogen peroxide and hot sterile air are supplied through special nozzles. As a result of mixing a fine mixture of peroxide and sterile hot air, a cloud of peroxide vapor with a concentration of peroxide is formed inside the monoblock, which makes it possible to destroy microorganisms and ensure complete sterility of the process of filling the product into containers and sterile sealing of containers. For the processing of containers and lids, nozzles are installed inside the monoblock, through which a sprayed fine mixture is fed, which is partially deposited on the walls of the container and the walls of the lids, partially mixed with the peroxide vapor available inside the monoblock. The peroxide deposited on the walls of containers and lids kills the remnants of microorganisms, ensures the sterility of the original packaging before bottling and capping. Immediately before spilling, the container is purged with a jet of hot sterile air to remove peroxide residues from the inner walls of the container. IImmediately before spilling, the container is purged with a jet of hot sterile air to remove peroxide residues from the inner walls of the container. Purging with hot sterile air is carried out by injectors installed inside the monoblock on the way of movement of containers and lids. As a result of exposure to hot air, the peroxide deposited on the inner walls of the container and the inner walls of the lid decomposes into water and oxygen, which reduces the concentration of residual peroxide in the product in the sealed container to acceptable values (below 0.5 ppm).

The results of the conducted tests on disinfection and filling of bottles reflect the organoleptic quality indicators of the process that ensures the production of a competitive finished product, the unit of measurement of the concentration of which (residual peroxide level) is equal to 0.3 ppm, which is lower than the generally accepted world standards of 0.5 ppm.

Thus, the analysis and testing of the prototype confirm the achievement of the stated technical result of the claimed invention: increasing reliability, simplifying and reducing the cost of the bottling process by creating a hydrogen peroxide sterilizer of a reasonable structure that provides the required high biological purity of the bottled beverage occurring in.

The invention proposed for patenting has the criterion of "novelty", since the whole set of features of the formula is not known from the prior art given in the corresponding section of the description.

It has the criterion of "inventive level", since for a specialist it does not explicitly follow from the state of the art.

And it is also industrially applicable, since the tests carried out have confirmed the possibility of its use in the presented field of technology.

## Claims

1. The beverage bottling plant, **characterized by** being a sterile leaky insulator, the inner space of which is filled with a sterilizing mixture including hot air and atomized fine hydrogen peroxide - H2O2 - peroxide vapor, is made according to the principle of a single-circuit insulator housing starting from the entrance of the container into the sterile space of the filling machine and until the exit (6) of the already sealed container with the product, it operates in a continuous mode, at the inlet and outlet of the container there are hoods (2, 3) of the hood, which create a controlled movement of the sterilizing medium inside, while the hoods of the hood provide a constant ejection effect, thanks to which they suck partially peroxide vapor and partially air from the surrounding space, with the right flow rate of the hoods, such a movement of the sterilizing medium is created inside, which eliminates the ingress of peroxide vapor outside through the leaky skin, the air entering through the leaky seals of the space and through the feed channel of the covers immediately mixes with peroxide vapor and becomes sterile, this eliminates the ingress of microorganisms into the sealed container, peroxide vapor inside is formed by feeding (4, 5) inside a sprayed fine mixture with a high concentration of peroxide for processing incoming containers inside and outside and for processing lids for sealing containers, as well as by feeding inside sterile hot air to neutralize peroxide on the inner walls of the container and on the inner walls of the lids, this eliminates the ingress of microorganisms into the sealed container, peroxide vapor inside is formed by feeding inside a sprayed fine mixture with a high concentration of peroxide for processing incoming containers inside and outside and for processing lids for sealing containers, as well as by feeding inside sterile hot air to neutralize peroxide on the inner walls of the container and on the inner walls of the lids this eliminates the ingress of microorganisms into the sealed container, peroxide vapor inside is formed by feeding inside a sprayed fine mixture with a high concentration of peroxide for processing incoming containers inside and outside and for processing lids for sealing containers, as well as by feeding inside sterile hot air to neutralize peroxide on the inner walls of the container and on the inner walls of the lids, for the treatment of containers and lids, nozzles (7-11) are installed inside, through which a sprayed fine mixture is fed, which is partially deposited on the walls of the container and the walls of the lids, partially mixed with the peroxide vapor available inside the monoblock (1), the peroxide deposited on the walls of containers and lids kills the remnants of microorganisms, ensures the sterility of the original packaging before bottling and capping, immediately before spilling, the container is purged with a jet of hot sterile air to remove peroxide residues from the inner walls of the container, immediately before spilling, the container is fired with a jet of hot sterile air to remove peroxide residues from the inner walls of the container, as a result of exposure to hot air, the peroxide deposited on the inner walls of the container and the inner walls of the lid decomposes into water and oxygen, which reduces the concentration of residual peroxide in the product in the sealed container to acceptable values.
